Europäisches Patentamt

European Patent Office

Office européen des brevets

(11) Veröffentlichungsnummer: **0 468 199 A1**

# EUROPÄISCHE PATENTANMELDUNG

(21) Anmeldenummer: **91110149.1**

(22) Anmeldetag: **20.06.91**

(51) Int. Cl.5: **C08G 63/08, A61K 47/48**

(30) Priorität: **23.06.90 EP 90111954**
**11.09.90 DE 4028764**

(43) Veröffentlichungstag der Anmeldung:
**29.01.92 Patentblatt 92/05**

(84) Benannte Vertragsstaaten:
**AT BE CH DE DK ES FR GB GR IT LI LU NL SE**

(71) Anmelder: **BOEHRINGER INGELHEIM KG**

**W-6507 Ingelheim am Rhein(DE)**

(84) **AT BE CH DE DK ES FR GB GR IT LI LU NL SE**

Anmelder: **BOEHRINGER INGELHEIM INTERNATIONAL G.M.B.H.**

**W-6507 Ingelheim am Rhein(DE)**

(84) **GB**

(72) Erfinder: **Stricker, Herbert, Prof.Dr.**
**Richard-Lenel-Weg 13**
**W-6903 Neckargemünd(DE)**
Erfinder: **Bendix, Dieter, Dr. Dipl.-Chem.**
**Veith-Stoss-Strasse 17**
**W-6507 Ingelheim am Rhein(DE)**

(54) **Verfahren zur Herstellung von Poly-(D,L-lactid) und seine Verwendung als Wirkstoffträger.**

(57) D,L-Lactid wird in Gegenwart von Katalysatoren und definierter Mengen Polymilchsäure zu Poly(D,L-lactid) polymerisiert. Das vorzugsweise niedermolekulare Produkt soll als implantierbarer Träger pharmakologischer Wirkstoffe eingesetzt werden.

EP 0 468 199 A1

Die Erfindung betrifft ein neues Verfahren zur Herstellung niedermolekularer Poly-D,L-lactide sowie deren Verwendung als Wirkstoffträger für Arzneimittel.

Aus dem Stand der Technik sind zahlreiche Wirkstofffreigabesysteme auf Basis resorbierbarer Polymere bekannt, die injiziert oder implantiert werden. Solche Systeme werden immer dann bevorzugt, wenn der Wirkstoff über einen längeren Zeitraum freigesetzt werden soll und eine perorale Applikation nicht möglich bzw. nicht ausreichend verläßlich bzw. wirksam ist. Ein wirkstoffhaltiges resorbierbares Implantat ist gegenüber der oralen Applikation auch dann bevorzugt, wenn der Wirkstoff im Körper innerhalb eines definierten räumlichen Bereichs abgegeben werden soll, z.B. hochwirksame Cytostatica in Tumoren.

Implantierbare Wirkstoffträger sollten folgende Kriterien erfüllen:

Der Wirkstoff sollte über längere Zeit in gleichbleibender Geschwindigkeit abgegeben und der Polymerträger in einem angemessenen Zeitraum abgebaut werden, so daß eine operative Entfernung des Implantats nach der Wirkstofffreigabe entfällt. Wichtige Parameter sind in diesem Zusammenhang die Quellfähigkeit (Wasseraufnahme), die Molmassenabnahme und die Massenabnahme des Polymeren.

Verfahren zur Herstellung von Poly-D,L-lactid - z.B. durch Polymerisation von Lactid in Gegenwart geeigneter Polymerisationskatalysatoren (höhere Molmassen) oder durch Polykondensation von Milchsäure (niedrige Molmassen) - gehören zum Stand der Technik.

Unter "Polymilchsäure" werden hier Polymere verstanden, die aus Milchsäureeinheiten aufgebaut sind und einen niedrigen Polymerisationsgrad aufweisen. Solche Polymilchsäuren werden üblicherweise durch Kondensation von Milchsäuren hergestellt, werden aber auch bei der ringöffnenden Polymerisation von Lactiden unter geeigneten Bedingungen erhalten.

Überraschenderweise wurde gefunden, daß ein implantierbarer Wirkstoffträger, der aus einem Poly-D,L-lactid mittlerer Molmasse, das nach dem folgenden Verfahren hergestellt wird, besondere Vorteile aufweist.

Das erfindungsgemäße Verfahren ist dadurch charakterisiert, daß D,L-Lactid in Gegenwart geeigneter Polymerisationskatalysatoren unter Zusatz definierter Anteile von Polymilchsäure polymerisiert wird.

Geeignete Polymerisationskatalysatoren sind aus dem Stand der Technik bekannt, so z.B. organische und anorganische Zinnverbindungen, wie beispielsweise Zinnlactat, Zinntartrat, Zinnoxalat, Zinndicaprylat, Zinndilaurat, Zinndipalmitat, Zinnchlorid u.a.. Besonders bevorzugt ist Zinndioctoat, besser bezeichnet als Zinn-di-(2-ethyl-hexanoat).

Der Zusatz an Polymilchsäure - bevorzugt Poly-D,L-milchsäure - liegt zwischen 5 und 40, vorzugsweise zwischen 20 und 30 %, bezogen auf das eingesetzte D,L-Lactid. Erfindungsgemäß wird unter Polymilchsäure: Poly-D-, Poly-L- oder Poly-D,L-milchsäure verstanden. Die eingesetzte Polymilchsäure sollte ein mittleres Molekulargewicht, bestimmt durch Endgruppentitration, von ca. 1800 bis 2500 (Zahlenmittel) besitzen.

Das Gewichtsmittel, bestimmt durch Gelpermeationschromatographie gegen engverteilte Polystyrol-Standards, sollte zwischen 3000 and 4000 liegen, die Polydispersität bei ca. 2,5 bis 3.5.

Nach dem erfindungsgemäßen Verfahren hergestelltes Poly-D,L-lactid (inhärente Viskosität (i.V.) - gemessen in Chloroform bei 25 °C, $c = 100$ mg/100 ml - zwischen 0.15 und 0.25, bevorzugt 0.17-0.20 dl/g, gewichtsmittlere Molmasse (Mw) - bezogen auf engverteilte Polystyrolstandarts in Chloroform - zwischen $1.10^4$ und $1.4 . 10^4$, bevorzugt $1.2 . 10^4$, Polydispersität D: 1,7 bis 2,0) eignet sich zur Herstellung der erfindungsgemäßen implantierbaren Wirkstoffträger.

Nach dem erfindungsgemäßen Verfahren hergestellte Polymere und daraus gefertigte Implantate weisen folgende Vorteile auf:

1) Schmelzverhalten:

niedriger Schmelzbereich, bevorzugt 60 - 75 °C. Daher niedrige Massetemperatur bei der Verarbeitung mittels Extrusion.

2) Glasübergangstemperatur (Tg):

Die erfindungsgemäß hergestellten Polymere behalten ca. 30 Tage lang einen Tg von ca. 45 °C, d.h. über der Körpertemperatur. Implantate aus diesen Polymeren behalten diese Zeit den ursprünglichen mechanischen Zustand und gewährleisten so konstante Freigabebedingungen.

3) Quellverhalten:

Im Gegensatz zu Poly-(D,L-lactid-co-glycolid) beginnt das erfindungsgemäß hergestellte Poly-D,L-lactid praktisch erst nach ca. 35 Tagen mit einer stärkeren Wasseraufnahme. Bei Poly-(D,L-lactid-co-glycolid) (Molverhältnis: 1:1, i.V. ca. 0,4) tritt dies bereits nach ca. 8 Tagen ein. Diese Eigenschaft spielt für die Stabilität inkorporierter, hydrolyseempfindlicher Wirkstoffe eine große Rolle.

4) Molmassenabnahme:

Die erfindungsgemäß hergestellten Polymere zeigen erst nach ca. 10 Tagen eine signifikante Molmassenabnahme und sind demnach besser zur Herstellung von Freigabesystemen mit konstantem Freigabeverhalten geeignet. Für die Wirkstofffreigabe ist von großer Bedeutung, daß bei einen Körper, z.B. einem Inplantat, der Polymerabbau im Inneren rascher erfolgt als an der dem wässerigen Medium ausgesetzten Oberfläche. Dadurch entsteht eine Barriere, deren Permeabilität die Freigabe der inkorporierten Wirkstoffe und Polymerabbauprodukte kontrolliert.

5) Masseabnahme:

Ca. 35 Tage lang nach Einbringen in Phosphatpuffer (isoton, pH 7,4; 37 °C) erfolgt keine Massenabnahme, danach kommt es innerhalb von ca. 40 Tagen zum vollständigen Polymerabbau zu Milchsäure.

Die Implantate aus den erfindungsgemäß hergestellten Polymeren können nach verschiedenen Verfahren hergestellt werden, so z.B. durch Extrusion (I) oder nach der Lösungsmethode (II).

Zu I: Poly-D,L-lactide mit inhärenten Viskositäten zwischen 0.15 und 0.25 weisen einen niedrigen Schmelzbereich auf und können somit mit vielen Arzneistoffen ohne deren thermische Zersetzung mittels Extrusion verarbeitet werden. Hierzu wird das Polymer in Form eines Pulvers oder Granulats mit dem Wirkstoff vermischt und brikettiert. Falls gewünscht, können weitere Hilfsstoffe, z.B. wasserlösliche Porenbildner wie Lactose, zugesetzt werden. Anschließend wird die Mischung extrudiert und zu Stücken (z.B. Zylinder, Durchmesser 2mm, Länge 2 cm) geschnitten.

Zu II: Bei der Lösungsmethode werden das Polymer und eventuelle Hilfsstoffe - wie Weichmacher - in einem geeigneten Lösungsmittel gelöst, zu Filmen oder in Formen gegossen, getrocknet und weiterverarbeitet. Hierbei sind folgende Ausführungsformen denkbar:

A) massive Stäbchen,

B) gerollte Filme,

C) ummantelte Stäbchen,

D) schlauchförmige Körper,

E) ummantelte schlauchförmige Körper

Alle Ausführungsformen des erfindungsgemäßen Implantats können mehrschichtig aufgebaut sein. Beispielsweise kann die Form A nach folgendem Verfahren gefertigt werden:

In der Polymerlösung (z.B. aus Essigester, Aceton oder einem anderen pharmakologisch unbedenklichen Lösungsmittel), wird der Wirkstoff gelöst oder suspendiert. Wenn erforderlich, können neben dem Wirkstoff pharmazeutische Hilfsstoffe, wie z.B. wasserlösliche Porenbildner oder Weichmacher, zugesetzt werden. Danach wird die Lösung oder Suspension auf einer definierten Fläche ausgegossen und im Vakuum bis zu einen Lösungsmittelrestgehalt von bevorzugt 3 bis 6 % getrocknet. Geräte und Verfahren zur Herstellung solcher Filme sind dem Fachmann bekannt und bedürfen keiner weiteren Ausführungen. Mehrschichtige Gießlinge werden durch ein wiederholtes Aufbringen von Polymerlösung (mit oder ohne Wirkstoff) erhalten. Die bevorzugte Schichtdicke liegt im Bereich 200 - 300 $\mu$m.

Schließlich werden unter aseptischen Bedingungen Stäbchen der gewünschten Breite und Länge geschnitten.

Rollen des Typs B bestehen aus ein- oder mehrschichtigen Polymerfilmen, wobei der Wirkstoff nur in einer oder in allen Schichten enthalten sein kann und die Dicke der Filme, im allgemeinen zwischen 30 und 500 $\mu$m, bevorzugt 70 bis 90 $\mu$m beträgt. Nach dem Trocknen werden die Filme geschnitten und zu Rollen gewünschter Abmessungen z.B. mit 3 cm Länge geformt. Durch die Verbindung mehrerer Filmschichten können in einfacher Weise Wirkstoffe kombiniert werden und definierte Konzentrationsprofile hergestellt werden, so daß die einzelnen Schichten verschiedene Freisetzungsgeschwindigkeiten aufweisen.

Beispiel 1:

In ausgeheizten Zweihalskolben wurden im Handschuhkasten unter trockenem Stickstoff jeweils 40, 35 und 30 g D,L-Lactid, sowie 10 (20 %), 15 (30 %) und 20 g (40 %) Poly-(D,L-milchsäure) eingewogen. Die zugesetzte Polymilchsäure (PMS) hatte folgende analytischen Daten:

```
Mn = 2000              (Endgruppentitration)

Mn = 1100              (GPC, ND-PS Standards)

Mw = 3400              (GPC, ND-PS Standards)

Gehalt an D,L-Lactide: 2 %   (NMR, CDCl3, 250 MHz)

Wassergehalt:          0,2%  (KF-Titration)
```

Unter trockenem Stickstoff wurde das Gemisch bei 130 °C aufgeschmolzen und dann 25.6 mg Zinnoctoat in toluolischer Lösung als Katalysator zugegeben. Jede Stunde wurde eine Probe gezogen und die inhärente Viskosität (IV) bestimmt
(c = 0.1g/100 ml, Chloroform, 25 °C).

| Reaktions-zeit [h] | 20 %PMS IV [dl/g] | 30 % PMS IV [dl/g] | 40 % PMS IV [dl/g] |
|---|---|---|---|
| 1 | .08 | .07 | .08 |
| 2 | .12 | .09 | .07 |
| 3 | .16 | .11 | .09 |
| 4 | .18 | .13 | .09 |
| 5 | .20 | .14 | .10 |
| 6 | .21 | .15 | .11 |
| 7 | .22 | .15 | .11 |

Beispiel 2:

Im Handschuhkasten wurden unter trockenem Stickstoff 400 g D,L-Lactid und 100 g Poly(D,L-milchsäure) in einem Kolben eingewogen.
Das Gemisch wurde unter Rühren und Überdeckung mit trockenem Stickstoff bei 140 °C aufgeschmolzen, die Ölbadtemperatur auf 130 °C gesenkt und 256 mg Zinnoctoat in toluolischer Lösung als Katalysator zugegeben. Die nach 5 Stunden Polymerisation erhaltene glasartige Polymerschmelze wurde analysiert:

Inhärente Viskosität: 0.17 dl/g
Restmonomergehalt: 10.5 % (NMR, $CDCl_2$, 250 MHz)
$M_w$ 13 500 (GPC, PS-Standards, $CHCl_3$)
$M_n$ 6 200 (GPC, PS-Standards, $CHCl_3$)
Polydispersität Mw/Mn 2,2

Das Polymer wurde anschließend in der vierfachen Menge Aceton unter kräftigem Rühren in der Siedehitze gelöst, aus Wasser umgefällt und im Vakuumtrockenschrank bei 30 °C getrocknet.
Inhärente Viskosität: 0.18 dl/g

Beispiel 3:

In einem helicalen Mixer wurden 7.5 kg D,L-Lactid und 2.5 kg Poly(D,L-milchsäure) vorgelegt und unter Stickstoff aufgeschmolzen. 5 g Zinnoctoat in 20 ml Toluol wurden zugegeben und 5 Stunden bei 170 °C Ölbadtemperatur polymerisiert.
Ausbeute: 98 % d. Einsatzes
Das Polymer wurde in 50 l Aceton in der Siedehitze gelöst, filtriert, aus Wasser umgefällt und im Vakuumtrockenschrank bei 38 °C getrocknet.
Ausbeute: 77 % d.E.
Inhärente Viskosität: 0.19 dl/g
Restmonomergehalt: << 1 % (NMR, $CDCl_3$, 250 MHz)

**Patentansprüche**

**1.** Verfahren zur Herstellung von Poly-D,L-lactid durch ringöffnende Polymerisation von D,L-Lactid in

4

Gegenwart von Polymerisationskatalysatoren, gekennzeichnet durch einen Zusatz definierter Anteile von Polymilchsäure.

2. Verfahren nach Anspruch 1, gekennzeichnet durch einen Anteil von 20 bis 30 Gew.-%. Polymilchsäure bezogen auf eingesetztes D,L-Lactid.

3. Verfahren nach Anspruch 1 oder 2, gekennzeichnet durch einen Anteil von 20 bis 30 Gew.-% Poly-D,L-milchsäure.

4. Poly-D,L-lactid hergestellt durch ringöffnende Polymerisation von D,L-Lactid in Gegenwart von Polymerisationskatalysatoren unter Zusatz von Polymilchsäure.

5. Poly-D,L-lactid nach Anspruch 4 gekennzeichnet durch einen Zusatz an Poly-D,L-milchsäure.

6. Poly-D,L-lactid nach Anspruch mit einer inhärenten Viskosität zwischen 0.15 und 0.25 dl/g (gemessen in Chloroform bei 25°C).

7. Implantierbarer Wirkstoffträger auf der Basis von Poly-D,L-lactid gemäß Anspruch 4, 5 oder 6, enthaltend mindestens einen pharmakologisch aktiven Wirkstoff sowie gegebenenfalls übliche Hilfs- und Trägerstoffe.

**Europäisches Patentamt**

# EUROPÄISCHER RECHERCHENBERICHT

Nummer der Anmeldung

## EP 91 11 0149

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | WO-A-9 001 521 (BATTELLE MEMORIAL INST.)<br>* Seite 10, Zeile 31 - Seite 12, Zeile 5; Seite 46, Zeile 22 - Seite 47, Zeile 6; Seite 58, Zeile 30 - Seite 59, Zeile 19 *<br>– – – | 1-6 | C 08 G 63/08<br>A 61 K 47/48 |
| A | DE-A-2 827 289 (ETHICON INC.)<br>* Anspruch 1; Beispiele 4,5 *<br>– – – | 1 | |
| A | US-A-3 887 699 (S. YOLLES)<br>* Spalte 1, Zeilen 11-16; Spalte 3, Zeile 66 - Spalte 4, Zeile 25; Spalte 5, Zeilen 27-42; Spalte 5, Zeile 61 - Spalte 6, Zeile 9; Spalte 8, Zeile 46 - Spalte 9, Zeile 39; Spalte 10, Zeilen 32-45 *<br>– – – – – | 7 | |

**RECHERCHIERTE SACHGEBIETE (Int. Cl.5)**

C 08 G

Der vorliegende Recherchenbericht wurde für alle Patentansprüche erstellt

| Recherchenort | Abschlußdatum der Recherche | Prüfer |
|---|---|---|
| Den Haag | 31 Oktober 91 | KRISCHE D.H.T. |